Europäisches Patentamt

European Patent Office

Office européen des brevets

(19)

(11) **EP 1 042 496 B1**

(12) **FASCICULE DE BREVET EUROPEEN**

(45) Date de publication et mention
de la délivrance du brevet:
**19.03.2003 Bulletin 2003/12**

(21) Numéro de dépôt: **98963586.7**

(22) Date de dépôt: **22.12.1998**

(51) Int Cl.7: **C12P 7/42**, C12N 9/02

(86) Numéro de dépôt international:
**PCT/FR98/02819**

(87) Numéro de publication internationale:
**WO 99/034008 (08.07.1999 Gazette 1999/27)**

(54) **PROCEDE DE PREPARATION ENZYMATIQUE D'HOMOGENTISATE**

ENZYMATISCHE HERSTELLUNG VON HOMOGENTISINSÄURE

METHOD FOR ENZYMATIC PREPARATION OF HOMOGENTISATE

(84) Etats contractants désignés:
**AT BE CH DE DK ES FI FR GB GR IE IT LI LU NL
PT SE**

(30) Priorité: **24.12.1997 FR 9716727**

(43) Date de publication de la demande:
**11.10.2000 Bulletin 2000/41**

(73) Titulaire: **Bayer CropScience S.A.
69009 Lyon (FR)**

(72) Inventeurs:
 • **DEROSE, Richard
 F-91000 Evry (FR)**
 • **SAILLAND, Alain
 F-69009 Lyon (FR)**

(74) Mandataire: **Mérigeault, Shona et al
Aventis CropScience SA
Département Propriété Industrielle
14-20 rue Pierre Baizet
B.P. 9163
69263 Lyon Cedex 09 (FR)**

(56) Documents cités:
 **EP-A- 0 343 330        WO-A-93/08295**

 • **CHEMICAL ABSTRACTS, vol. 123, no. 15, 9
   octobre 1995 Columbus, Ohio, US; abstract no.
   193271, SUEMORI, AKIO ET AL:
   "L-Phenylalanine and L-tyrosine degradative
   pathways in Rhodococcus erythropolis"
   XP002079481 & SEIMEI KOGAKU KOGYO
   GIJUTSU KENKYUSHO KENKYU HOKOKU
   (1995), 3(2), 33-6 CODEN: SKGIEM;ISSN:
   0919-5351,1995,**
 • **BLAKLEY E R: "The catabolism of L-tyrosine by
   an Arthrobacter sp." CANADIAN JOURNAL OF
   MICROBIOLOGY, vol. 23, 1977, pages
   1128-1139, XP002079478 cité dans la demande**
 • **SUEMORI A ET AL: "Purification and
   characterisation of o-hydroxyphenylacetate
   5-hydroxylase, m-hydroxyphenylacetate
   6-hydroxylase and p-hydroxyphenylacetate
   1-hydroxylase from Rhodococcus erythropolis."
   JOURNAL OF FERMENTATION AND
   BIOENGINEERING, vol. 81, no. 2, 1996, pages
   133-137, XP002079479 cité dans la demande**
 • **HARELAND W A ET AL: "Metabolic function and
   properties of 4-hydroxyphenylacetic acid
   1-hydroxylase from Pseudomonas
   acidovorans." JOURNAL OF BACTERIOLOGY,
   vol. 121, no. 1, 1975, pages 272-285,
   XP002079480 cité dans la demande**

**Description**

La présente invention concerne un nouveau procédé de préparation enzymatique d'homogentisate, ou acide 2,5-dihydroxy-phényl acétique (ci-après HMO).

L'HMO est un précurseur connu de molécules appelées les pigments ochronotiques qui sont des analogues de la mélanine. Ces molécules brunes sont d'ailleurs souvent citées comme "mélanine like pigments", qui trouvent une application variée dans la cosmétique ou l'industrie pharmaceutique. L'addition de mélanine ou mélanine like pigments dans les laits solaires auraient un effet protecteur intéressant. Pour produire ces dérivés ochronotiques à partir d'HGA le procédé est simple puisque la molécule s'auto-oxyde rapidement en conditions alcalines. Différents procédés de préparation enzymatique d'HMO à partir d'acide 1-phényl acétique ou de tyrosine sont décrits dans l'état de la technique (WO 93/08295 ou EP 343 330), de même que la préparation subséquente de mélanine.

On sait également que l'HMO est un composé essentiel à la vie des plantes. Dans les cellules végétales, l'HMO est le produit de transformation enzymatique du 4-hydroxyphénylpyruvate (ci-après HPP) par la 4-hydroxyphénylpyruvate dioxygénase (ci-après HPPD). Les inhibiteurs de cette enzyme sont des composés herbicides qui bloquent la production d'HMO dans la cellule végétale (Pallett K. E. et *al.* 1997 Pestic. Sci. <u>50</u> 83-84). Lorsque l'on fait germer des plantes, d'*Arabidopsis thaliana* par exemple, sur milieu synthétique en présence d'un inhibiteur de l'HPPD, les plantes vont germer, rester blanches puis mourir très rapidement. Or, si dans le milieu synthétique additionné d'un inhibiteur de l'HPPD, on ajoute de l'HMO les plantes vont germer normalement et rester vertes tant que le milieu contiendra de l'HMO. Il est donc également important de pouvoir disposer d'HMO pour prévenir les carences des plantes liées à un dysfonctionnement métabolique de la biosynthèse de l'HMO, naturel ou induit notamment par des inhibiteurs de l'HPPD.

La présente invention concerne donc un procédé de préparation enzymatique d'HMO à partir de l'HPP, et plus particulièrement un procédé de préparation enzymatique d'HMO à partir de l'HPP insensible aux inhibiteurs de l'HPPD.

Le procédé selon l'invention consiste à effectuer dans un milieu réactionnel approprié les réactions enzymatiques suivantes:

- conversion enzymatique du HPP en 4-hydroxyphénylacétate (ci-après HPA) par une HPP-oxydase, puis
- conversion enzymatique de HPA en HMO par une HPA-hydroxylase .

La première réaction enzymatique suivante

4-hydroxyphénylpyruvate (HPP) → 4-hydroxyphénylacétate (HPA)

est catalysée par une HPP-oxydase. De telles oxydases se trouvent dans de nombreuses espèces procaryotiques ou eucaryotiques, en particulier dans les bactéries capables de pousser sur l'HPP comme seule source de carbone, le transformant en HPA , plus particulièrement chez un *Arthrobacter* où une telle oxydase responsable d'une étape du catabolisme de la tyrosine (Blakley,E.R. 1977 Canadian Journal of Microbiology <u>23</u> 1128-1139).

La deuxième réaction enzymatique suivante :

4-hydroxyphénylacétate (HPA) → homogentisate (HMO)

est catalysée par une HPA-hydroxylase. De telles hydroxylases se trouvent dans de nombreuses espèces procaryotiques ou eucaryotiques, en particulier dans les bactéries capables de pousser sur l'HPA comme seule source de carbone, le transformant en HMO, plus particulièrement chez *Pseudomonas acidovorans*, souvent appelée *Comamonas acidovorans*. (Hareland, W.A. et al 1975 Journal of Bacteriology <u>121</u> 272-285) chez certaines *Xanthobacter* (Van Den Tweel W. J. J. et *al*. 1986 Antonie van Leeuwenhoek <u>52</u> 309-318), chez *Pseudomonas alcaligenes* (Karigar C. S. et Pujar B. G. 1993 FEMS Microbiology Letters <u>110</u> 59-64), chez *Flavobacterium sp* (Van Den Tweel W. J. J. et *al*. 1988 Arch Microbiol. <u>149</u> 207-213), chez *Bacillus subtillis* (Crawford R. L. 1978 FEMS Microbiology Letters <u>4</u> 233-234), chez *Nocardia* sp DM1 (Raju S. G. et Vaidyanathan C. S. 1986 J.Indian Inst. Sci. <u>66</u> 511-520) et chez *Rhodococcus erythropolis* (Suemori A. et *al*. 1996 Journal of Fermentation And Bioengineering Vol 81, N°2 133-137). En outre, Suemori et al. (1995, Report of National Institute of Bioscience and Human Technology - Seimei Kogaku Kogyo Gijutsu Kenkyusho Kenkyu Hokoku 3(2), 33-36) rapporte l'existence d'une HPA-hydroxylase de *Rhodococcus erythropolis*, mais n'indique ni ne suggère aucune enzyme susceptible catalyser la conversion de l'HPP en HPA.

De manière avantageuse, l'HPA-hydroxylase employée dans le procédé selon l'invention est extraite de *Pseudomonas acidovorans*.

# EP 1 042 496 B1

Selon un mode préférentiel de réalisation de l'invention, les deux réactions enzymatiques sont effectuées dans le même milieu réactionnel contenant de l'HPP, les enzymes HPP-oxydase et HPA-hydroxylase étant présentes ensemble simultanément dans le milieu réactionnel.

Les enzymes HPP-oxydase et HPA-hydroxylase peuvent être introduites dans le milieu réactionnel approprié sous forme d'extrait protéique, ledit extrait protéique pouvant être brut ou purifié totalement ou en partie, ou encore produites in situ par des organismes biologiques appropriés. Elles peuvent être ainsi produites in situ par chaque organisme biologique produisant naturellement les deux enzymes, ou encore par un seul organisme biologique modifié de manière qu'il produise les deux enzymes. Cet organisme biologique peut être une bactérie, une levure ou encore une cellule végétale.

Les deux enzymes étant insensibles aux inhibiteurs de l'HPPD, le procédé selon l'invention peut être conduit en présence d'un inhibiteur de l'HPPD dans le milieu réactionnel approprié.

Le milieu réactionnel approprié est constitué par tout milieu aqueux dont les conditions de température, de pH et de force ionique sont appropriés pour les réactions enzymatiques. Lorsque les enzymes sont produites in situ par un ou plusieurs organismes biologiques, le milieu réactionnel est approprié pour la croissance desdits organismes.

En fin de réaction, l'HMO peut être isolé du milieu réactionnel et purifié, ou bien laissé dans le milieu réactionnel. Dans ce deuxième cas, le milieu réactionnel contenant l'HMO peut être employé comme milieu nutritif pour la culture de plantes, plus particulièrement pour la culture de plantes présentant un dysfonctionnement métabolique de la biosynthèse de l'HMO, naturel ou induit notamment par des inhibiteurs de l'HPPD.

Les exemples ci-après permettent d'illustrer l'invention, sans toutefois chercher à en limiter la portée.

## Exemple 1: Production d'HPA à partir d'HPP avec un extrait protéique d'*Arthrobacter*

### Culture d'*Arthrobacter* :

*Arthrobacter globiformis* est cultivé à 28°C, 220 tours par minute pendant 20 heures en Erlenmeyer de 250 ml contenant 50 ml de milieu A supplémenté avec 0,1% L-tyrosine et 0,01 % d'extrait de levure [milieu A composé en gramme par litre de $KH_2PO_4$ (1,5) $K_2HPO_4.3H_2O$ (3,5) $NH_4NO_3$ (1) $FeSO_4.7H_2O$ (0,1) $ZnSO_4.7H_2O$ (0,01) $NaMoO_2.7H_2O$ (0,01) $CaCl_2.2H_2O$ (0.01) $MgSO_4.7H_2O$ (0,05)]..

### Extraction et dosage de l'activité HPP-oxidase.

A partir de 2,1 litres de culture un culot de cellules est récupérée par centrifugation à 3000 xg pendant 15 min. Le culot est lavé avec de l'eau distillé puis recentrifugé. Toutes les étapes ultérieures sont faites à 4°C.

Le culot de cellules, environ 3,4g est resuspendu dans 7 ml de tampon d'extraction (phosphate de potassium 0,05M pH7,5, TPP 0,1mM, $MgCl_2$ 0,1mM et mercaptoethanol 5mM) et soniqué à 23kHz pendant 2,5 min. 2 fois. La suspension résultante est centrifugée à 44 000 xg pendant 20 min. Le surnageant récolté est centrifugé à nouveau à 100 000 xg pendant 60 min. Le nouveau surnageant faisant 7 ml est additionné de 0, 7 ml de protamine sulfate 2% dans le tampon d'extraction suivi d'une agitation douce. Le précipité qui se forme est éliminé par une centrifugation à 20 000 xg pendant 20 min. Le surnageant ainsi obtenu est additionné graduellement de sulfate d'ammonium pour arriver à 60% de la saturation. Cette nouvelle préparation est agitée 30 min. et le précipité formé récolté par centrifugation à 20 000 xg pendant 20 min. Le culot est resolubilisé dans 0,5 ml de tampon d'extraction et aliquoté en fractions de 0,1ml, fractions gardées à -80°C avant usage.

L'activité HPP-oxidase est mesurée en plaque de microtitration 96 puits avec 200 µl de réaction par puits consistant en 149,8 µl de sodium phosphate 67 mM pH7,4, 10µl de glutathion 13,4mM, 10µl de MgCl2 67 mM, 10µl de TPP 26,7mM, 10µl de FAD 67µM, 0,2µl de protéine (soit environ 6µg) et 10 µl d'HPP 2,5mM.

Quand on fait un essai d'inhibition par un inhibiteur de l'HPPD on ajoute 2µl de 4-[4-trifluorométhyle-2-(méthylsulfonyl)-benzoyl]-5-cyclopropyl isoxazole 10mM dans un tampon phosphate contenant 20% DMSO.

La réaction est initiée par l'addition du substrat, l'HPP, elle se déroule à 30°C pendant 5 min. sous agitation. La réaction est arrêtée par addition de 33 µl d'acide perchlorique 25%.

La plaque est ensuite centrifugée à 2000 tours par minute pendant 15 min. et le surnageant analysé par HPLC. 50 µl du surnageant est injecté sur une colonne Spherisorb ODS2 équilibrée avec le tampon A (acétonitrile 5,5%, TFA 0,1%) au débit de 1,5ml/min.

Le programme d'élution utilisé est:

0 min.: 0% de tampon B (acétonitrile)
6 min.: 15% de tampon B
6,5 min.: 15% de tampon B
7 min.: 60 % de tampon B

8 min.: 60 % de tampon B
8,5 min.: 0 % de tampon B

La détection est faite à 276 nm.

Le HPA produit par l'extrait enzymatique à partir d'HPP est comparé à une référence constituée d'HPA commercial en terme de temps de rétention et pic d'absorption spectrale.

Résultats.

L'analyse HPLC montre que l'extrait protéique extrait d'*Arthrobacter globiformis* cultivée sur tyrosine comme source majeure de carbone, est capable de transformer l'HPP en HPA (la molécule produite comigre parfaitement avec l'HPA commercial de référence).

L'enzyme responsable de cette réaction n'est pas inhibée par 100 μM d'inhibiteur de l'HPPD.

**Exemple 2: Production d'HMO à partir d'HPA avec un extrait protéique de *Pseudomonas***

Culture de l'organisme.

*Pseudomonas acidovorans* est cultivé à 28°C, 220 tours par minute pendant 20 heures en Erlenmeyer de 250 ml contenant 50 ml de milieu B supplementé avec 0,15% HPA et 0,01 % d'acide nitrilotriacetique. [ milieu B composé en gramme par litre de $NaH_2PO_4$ (1) $K_2HPO_4.3H_2O$ (4,25) $NH_4Cl$ (2) $FeSO_4.7H_2O$ (0,012) $ZnSO_4.7H_2O$ (0,003) $MnSO_4.7H_2O$ (0,003) $CoSO_4.7H_2O$ (0.01) $MgSO_4.7H_2O$ (0,2).]

Extraction et dosage de l'activité HPA-hydroxylase.

A partir de 0,1 litres de culture un culot de cellules est récupérée par centrifugation à 7500 xg pendant 10 min. Le culot est lavé avec de l'eau distillé puis recentrifugé. Toutes les étapes ultérieures sont faites à 4°C.

Le culot de cellules, environ 0,5 g est resuspendu dans 1,5 ml de tampon d'extraction (phosphate de potassium 0,1 M pH7,2, DTE 1 mM, $MgMgSO_4$ 5mM) et soniqué à 23 kHz pendant 2,5 min. 2 fois. La suspension résultante est centrifugée à 44 000 xg pendant 20 min. Le surnageant récolté est centrifugé à nouveau à 100 000 xg pendant 60 min. Le nouveau surnageant est aliquoté en fractions de 0,1ml, fractions gardées à -80°C avant usage.

L'activité HPA-hydroxylase est mesurée en plaque de microtitration 96 puits avec 200μl de réaction par puits consistant en 150 μl de sodium phosphate 0,1M pH7,2, 10μl de DTE 20 mM, 10μl de NADH 3 mM, 15μl de FAD 67μM, 10μl de protéine (soit environ 7μg) et 5 μl d'HPA 10 mM.

Quand on fait un essai d'inhibition par un inhibiteur de l'HPPD on ajoute 2μl de 4-[4-trifluorométhyle-2-(méthylsulfonyl)-benzoyl]-5-cyclopropyl isoxazole 10 mM dans un tampon phosphate contenant 20% DMSO.

La réaction est initiée par l'addition du substrat, l'HPA, elle se déroule à 30°C pendant 5 min. sous agitation. La réaction est arrêtée par addition de 33μl d'acide perchlorique 25%.

La plaque est ensuite centrifugée à 2000 tours par minute pendant 15 min. et le surnageant analysé par HPLC. 10μl du surnageant est injecté sur une colonne Spherisorb ODS2 équilibrée avec le tampon A (acétonitrile 5,5%, TFA 0,1%) au débit de 1,5ml/min.

Le programme d'élution utilisé est:

0 min.: 0% de tampon B (acétonitrile)
0,8 min.: 0% de tampon B
1 min.: 60% de tampon B
1,7 min.: 60 % de tampon B
1,9 min.: 0 % de tampon B
5 min.: 0 % de tampon B

La détection est faite à 292 nm.

Le HMO produit par l'extrait enzymatique à partir d'HPA est comparé à une référence constituée d'HMO commercial en terme de temps de rétention et pic d'absorption spectrale.

Résultats.

L'analyse HPLC a permis de montrer que l'extrait protéique extrait de *Pseudomonas acidovorans* est capable de transformer l'HPA en HMO (la molécule produite co-migre parfaitement avec l'HMO commercial de référence).

L'enzyme responsable de cette réaction n'est pas inhibée dans nos conditions de test par 100μM d'inhibiteur d'HPPD.

**Exemple 3: Production d'HMO à partir d'HPP avec un extrait protéique d'*Arthrobacter* et de *Pseudomonas***

L'activité HPP-oxidase couplée à l'activité HPA-hydroxylase

L'activité HPP-oxidase couplée à l'activité HPA-hydroxylase est mesurée en plaque de microtitration 96 puits avec 200μl de réaction par puits consistant en 100 μl de sodium phosphate 100 mM pH7,2, 10μl de DTE 20 mM, 10μl de NADH 3 mM, 15μl de FAD 67 μM, 10μl de gluthation 13,4 mM, 10μl de MgCl2 67 mM, 10μl de TPP 26,7 mM, 2μl d'extrait HPP-oxidase (soit environ 60 μg), 25μl d'extrait d'HPA-hydroxylase (soit environ 18 μg) et 10 μl d'HPP 10 mM.

La réaction est initiée par l'addition du substrat, l'HPP, elle se déroule à 30°C pendant 30 min sous agitation. La réaction est arrêtée par addition de 33 μl d'acide perchlorique 25%.

La plaque est ensuite centrifugée à 2000 tours par minute pendant 15 min. et le surnageant analysé par HPLC. 25μl du surnageant est injecté sur une colonne Spherisorb ODS2 équilibrée avec le tampon A (acétonitrile 5,5%, TFA 0,1%) au débit de 1,5ml/min.

Le programme d'élution utilisé est:

0 min.: 0% de tampon B (acétonitrile)
6 min.: 15% de tampon B
6,5 min.: 15% de tampon B
7 min.: 60 % de tampon B
8 min.: 60 % de tampon B
8,5 min.: 0 % de tampon B

La détection est faite à 276 nm et 292 nm simultanément

Résultats.

L'analyse HPLC a permis de montrer que l'extrait protéique extrait d'Arthrobacter globiformis associé à celui de *Pseudomonas acidovorans* est capable de transformer simultanément l'HPP en HMO (la molécule produite comigre parfaitement avec l'HMO commercial de référence).

**Revendications**

1.  Procédé de préparation enzymatique d'homogentisate (HMO) à partir de 4-hydroxyphénylpyruvate (HPP), **caractérisé en ce qu'**il consiste à effectuer dans un milieu réactionnel approprié les réactions enzymatiques suivantes:

    -   conversion enzymatique du HPP en 4-hydroxyphénylacétate (HPA) par une HPP-oxydase , puis
    -   conversion enzymatique de HPA en HMO par une HPA-hydroxylase.

2.  Procédé selon la revendication 1, **caractérisé en ce que** l'HPP-oxydase provient de bactéries capables de pousser sur l'HPP comme seule source de carbone.

3.  Procédé selon la revendication 1, **caractérisé en ce que** l'HPP-oxydase provient d'*Arthrobacter* .

4.  Procédé selon la revendication 1, **caractérisé en ce que** l'HPA-hydroxylase provient de bactéries capables de pousser sur l'HPA comme seule source de carbone.

5.  Procédé selon la revendication 4, **caractérisé en ce que** les bactéries sont choisies parmi *Pseudomonas acidovorans, Xanthobacter, Pseudomonas alcaligenes, Flavobacterium sp, Bacillus subtillis, Nocardia* sp DM1 et *Rhodococcus erythropolis*.

6.  Procédé selon la revendication 4, **caractérisé en ce que** l'HPA-hydroxylase est extraite de *Pseudomonas acidovorans*.

7.  Procédé selon l'une des revendications 1 à 6, **caractérisé en ce que** les deux réactions enzymatiques sont effectuées dans le même milieu réactionnel contenant de l'HPP, les enzymes HPP-oxydase et HPA-hydroxylase étant présentes ensemble simultanément dans le milieu réactionnel.

**8.** Procédé selon l'une des revendications 1 à 7, **caractérisé en ce que** les enzymes HPP-oxydase et HPA-hydroxylase sont introduites dans le milieu réactionnel approprié sous forme d'extraits protéiques, ou encore produites in situ par des organismes biologiques appropriés.

**9.** Procédé selon l'une des revendications 1 à 8, **caractérisé en ce qu'**il est réalisé en présence d'un inhibiteur de l'HPPD dans le milieu réactionnel approprié.

**Patentansprüche**

**1.** Verfahren zur enzymatischen Herstellung von Homogentisat (HMO) ausgehend von 4-Hydroxyphenylpyruvat (HPP), **dadurch gekennzeichnet, daß** es darin besteht, in einem geeigneten Reaktionsmedium die folgenden enzymatischen Reaktionen durchzuführen:

- enzymatische Umwandlung von HPP in 4-Hydroxyphenylacetat (HPA) durch eine HPP-Oxidase, und anschließend
- enzymatische Umwandlung von HPA in HMO durch eine HPA-Hydroxylase.

**2.** Verfahren nach Anspruch 1, **dadurch gekennzeichnet, daß** die HPP-Oxidase von Bakterien stammt, die befähigt sind, auf HPP als einziger Kohlenstoffquelle zu waschen.

**3.** Verfahren nach Anspruch 1, **dadurch gekennzeichnet, daß** die HPP-Oxidase von *Arthrobacter* stammt.

**4.** Verfahren nach Anspruch 1, **dadurch gekennzeichnet, daß** die HPA-Hydroxylase von Bakterien stammt, die befähigt sind, auf HPA als einziger Kohlenstoffquelle zu waschen.

**5.** Verfahren nach Anspruch 4, **dadurch gekennzeichnet, daß** die Bakterien unter *Pseudomonas acidovorans, Xanthobacter, Pseudomonas alcaligenes, Flavobacterium sp, Bacillus subtillis, Nocardia sp* DM1 und *Rhodococcus erythropolis* ausgewählt sind.

**6.** Verfahren nach Anspruch 4, **dadurch gekennzeichnet, daß** die HPA-Hydroxylase aus *Pseudomonas acidovorans* gewonnen wird.

**7.** Verfahren nach einem der Ansprüche 1 bis 6, **dadurch gekennzeichnet, daß** die beiden enzymatischen Reaktionen in dem gleichen Reaktionsmedium durchgeführt werden, das HPP und die Enzyme HPP-Oxydase und HPA-Hydroxylase enthält, die alle gleichzeitig in dem Reaktionsmedium vorhanden sind.

**8.** Verfahren nach einem der Ansprüche 1 bis 7, **dadurch gekennzeichnet, daß** die Enzyme HPP-Oxidase und HPA-Hydroxylase in Form von Proteinextrakten in das geeignete Reaktionsmedium gegeben werden oder in situ von geeigneten biologischen Organismen gebildet werden.

**9.** Verfahren nach einem der Ansprüche 1 bis 8, **dadurch gekennzeichnet, daß** es in Gegenwart einer HPPD-Inhibitors in dem geeigneten Reaktionsmedium durchgeführt wird.

**Claims**

**1.** Method for enzymatic preparation of homogentisate (HMO) from 4-hydroxyphenylpyruvate (HPP), **characterized in that** it consists in carrying out, in a suitable reaction medium, the following enzymatic reactions:

- enzymatic conversion of HPP into 4-hydroxyphenylacetate (HPA) with an HPP-oxidase, then
- enzymatic conversion of HPA into HMO with an HPA-hydroxylase.

**2.** Method according to Claim 1, **characterized in that** the HPP-oxidase originates from bacteria which can grow on HPP as the only carbon source.

3. Method according to Claim 1, **characterized in that** the HPP-oxidase originates from *Arthrobacter*.

4. Method according to Claim 1, **characterized in that** the HPA-hydroxylase originates from bacteria which can grow on HPA as the only carbon source.

5. Method according to Claim 4, **characterized in that** the bacteria are chosen from *Pseudomonas acidovorans, Xanthobacter, Pseudomonas alcaligenes, Flavobacterium sp., Bacillus subtillis, Nocardia sp.* DM1 and *Rhodococcus erythropolis*.

6. Method according to Claim 4, **characterized in that** the HPA-hydroxylase is extracted from *Pseudomonas acidovorans*.

7. Method according to one of Claims 1 to 6, **characterized in that** both enzymatic reactions are carried out in the same reaction medium containing HPP, the HPP-oxidase and HPA-hydroxylase enzymes being present together at the same time in the reaction medium.

8. Method according to one of Claims 1 to 7, **characterized in that** the HPP-oxidase and HPA-hydroxylase enzymes are introduced into the suitable reaction medium in the form of protein extracts, or alternatively they can be produced in situ by suitable biological organisms.

9. Method according to one of Claims 1 to 8, **characterized in that** it is carried out in the presence of an HPPD inhibitor in the suitable reaction medium.